# EUROPEAN PATENT APPLICATION

(11) **EP 1 518 576 A2**
(43) Date of publication of application: **30.03.2005**
(21) Application number: 04030848.8
(22) Date of filing: 23.10.2002
(51) Int. Cl.: A61M 5/32

(54) **Retractable safety needle**

(30) Priority: 24.10.2001 US 338910 P; 26.07.2002 US 399318 P; 15.10.2002 US 418787 P
(62) Divisional of application: 02023735.0
(71) Applicant: Becton, Dickinson and Company, Franklin Lakes, New Jersey 07417-1880 (US)
(72) Inventor: Saulenas, William G., Wayne New Jersey 07470 (US); Wilkinson, Bradley M., North Haledon New Jersey 07508 (US); Niermann, Volker, Bound Brook New Jersey 08805 (US); Swenson, Kirk D., North Caldwell New Jersey 07006 (US)
(74) Representative: Müller-Boré & Partner Patentanwälte

(57) **Abstract**

This invention refers to a retractable safety needle comprising:
a needle hub (36) having proximal and distal ends and a passage extending between said ends; a needle cannula (34) having a proximal end, a distal end and a lumen extending therebetween, said proximal end of said needle cannula (34) being securely mounted in said passage at said distal end of said needle hub (36); a resiliently deflectable actuator arm (60) extending from said needle hub (36), an actuator button being formed on said actuator arm (60) and projecting outwardly relative to said needle hub (36); a barrel (20, 22) having a proximal end, a distal end and a passage extending between said ends, said needle hub (36) being disposed in said passage of said barrel (20, 22) such that said needle hub (36), said needle cannula (34) and said actuator arm (60) are moveable relative to said barrel (20, 22) from a distal position where said needle cannula (34) projects distally beyond said barrel (20, 22) and a proximal position where said needle cannula (34) is disposed entirely in said barrel (20, 22), said barrel (20, 22) having an actuating opening extending therethrough, said actuator button projecting into said actuating opening and engaging portions of said barrel (20, 22) adjacent said actuating opening when said needle hub (36) is in said distal position, said actuator button being resiliently deflectable out of engagement with said actuating open; a spring (18) disposed in said barrel (20, 22) and adjacent said needle hub (36) for propelling said needle hub (36) to said proximal position when said actuator button is deflected out of said actuating aperture; and wherein said barrel (20, 22) includes an inwardly extending proximal flange (102) extending into said passage (98) at said proximal end of said barrel (20, 22) for engaging the flange (56) of the needle hub (36) and limiting proximal movement of said needle hub (36).

## Description

### 1. Field of the Invention

The invention relates to a medical apparatus with a piercing element.

### 2. Background of the Invention

Fluid collection sets, intravenous infusion sets and catheters are employed respectively for collecting bodily fluids from a patient, for infusing liquids into a patient. Fluid collection sets and intravenous infusion sets include a length of flexible plastic tubing with a proximal end connected to a plastic fitting and a distal end connected to a needle assembly. The needle assembly includes a hub and needle cannula. A pair of flexible plastic wings is mounted to or near the hub. The wings can be folded into face-to-face engagement with one another, and hence define a convenient handle for gripping and manipulating the needle cannula. The wings also can be rotated away from one another and can be taped into face-to-face contact with the skin of the patient. A catheter typically is used with an elongate piercing element for introducing the catheter into a patent.

Accidental sticks with a needle cannula can be painful and can transmit disease. As a result, most needle assemblies and other sharp medical implements are employed with rigid means for enclosing at least the sharp tip both prior to use and after use. Protection prior to use typically is achieved by a rigid plastic tube that has a proximal end frictionally mounted to or near the hub and a distal end that extends beyond the distal end of the piercing element. The plastic tube is removed and discarded immediately prior to use of the piercing element. Protection after use typically is achieved by a tubular shield that can be telescoped relative to both the hub and piercing element from a proximal position where the piercing element is exposed to a distal position where the piercing element is safely within the tubular shield. Shields of this type typically include means for releasably holding the shield in its proximal position and for holding the shield more securely in its distal position. Some devices include a spring for generating relative movement between the shield and the piercing element. In some instances, the shield is moved distally over the piercing element. In other instances, the piercing element is withdrawn proximally into the shield.

A small volume of blood or other bodily fluid may remain in or on a piercing element after the piercing element has been withdrawn from the patient. This residual fluid may splatter as the piercing element is retracted rearwardly into a shield. The probability of such splatter from a needle is dependent upon several factors, including the gauge of the needle, the acceleration of the needle in the proximal direction, the presence of any transverse movement of the needle during its rearward acceleration, the extent of capillary adhesion of the residual fluid on portions of the cannula that define the lumen and other factors. Splatter of bodily fluid can transmit disease.

Health care workers are required to use many different medical devices, and often use medical devices from several different manufacturers. The configuration and operation of the shields on the above-described medical devices vary widely from one manufacturer to another. A lack of familiarity with the specified operation of a shield for a particular medical device can lead to improper shielding and hence creates the potential for an accidental stick with a used needle cannula.

The ease of shielding and the effectiveness of the shielding also vary from one type of medical device to another. Devices that are mechanically simple may provide less effective shielding. More secure shielding may require more complex manipulation of the device by the health care worker. In view of the above, a demand exists for a medical device that provides secure shielding and an easy operation.

### Summary of the Invention

The present invention is a medical apparatus with a retractable piercing element. The apparatus may be a fluid collection or infusion set that comprises a length of flexible tubing with opposite proximal and distal ends and a passage extending between the ends. A fitting is secured to the proximal end of the flexible tubing and a needle assembly is secured to the distal end of the flexible tubing.

The piercing element may be a needle cannula having opposite proximal and distal ends and a lumen extending between the ends. The apparatus may include a hub with a proximal end, a distal end and a passage extending between the ends. A resiliently deflectable actuator arm is cantilevered from the hub and the free end of the cantilevered actuator arm is formed with an outwardly projecting actuator button. The proximal end of the piercing element is affixed to distal portions of the hub. Thus, a lumen that may be provided in the piercing element may communicate with the passage through the hub and with the passage through the flexible tubing. A rigid protector may be mounted removably over the piercing element and may extend sufficiently to cover the distal end of the piercing element.

The apparatus further includes a barrel telescoped over the hub such that the piercing element is movable relative to the barrel from a distal position to a proximal position. More particularly, the pointed distal end of the piercing element projects distally beyond the barrel when the piercing element is in the distal position relative to the barrel. However, all of the piercing element is safety enclosed within the barrel when the piercing element is in the proximal position relative to the barrel.

The barrel includes an actuation region that has an actuating aperture disposed and configured to permit engagement of the actuator button when the piercing element is in the distal position. Portions of the actuating region of the barrel may define a smaller cross-section than portions of the barrel either distally or proximally of the actuating region. Thus, the actuating aperture and the actuator button are recessed slightly relative to portions of the barrel on either longitudinal side of the actuating region to prevent inadvertent actuation that could displace the needle assembly relative to the barrel. Additionally, the recessed shape of the actuator region provides a visual cue to identify regions of the barrel that should be digitally accessed to retract the piercing element. This allows the user to locate the actuator button during use with or without visually seeing it and sometimes only relying on the change in tactile surface near the actuator region. The exterior shape of the barrel is preferably circular when viewed in a cross section perpendicular to the barrel's axis.

The barrel comprises means for preventing re-exposure of the piercing element after shielding has been effected. For example, the barrel may include an array of inwardly directed resilient fingers aligned to permit retraction of the piercing element relative to the barrel, but to prevent re-exposure of the retracted piercing element.

The apparatus further includes a pair of flexible wings. The wings preferably are formed separately from the barrel and are securely mounted to a portion of the barrel near the actuating region. Thus, the barrel may be formed from a first material selected for rigidity, while the wings may be formed from a second material selected for flexibility.

A lever may also be located on the barrel or in conjunction with the wings and in relation to the actuator button so as to activate the retractable piercing element.

The retractable apparatus further includes a spring for propelling the piercing element and hub proximally relative to the barrel. The spring may be biased to a collapsed condition when the piercing element is in its distal position relative to the barrel. However, disengagement of the actuator button from the actuating window permits the spring to expand and propels the piercing element and hub into its proximal position relative to the barrel.

The hub and barrel of the apparatus preferably are formed from translucent or transparent materials to provide "venous indication" or "flash" when the apparatus is part of a fluid collection set. This allows the user to identify when venous blood has reached the fluid path proximal from the proximal end of the needle cannula and to identify when venous blood has reached the inside of the needle hub.

The apparatus may further include a dampening agent, such as a viscous or non-viscous dampening agent to alter the acceleration and velocity of piercing element. Viscous dampening agents include grease, oil, gel, gel resin, or any combination thereof and non-viscous dampening agents include biased flexible elements extending between the front and rear barrels or the needle hub. Preferably, the dampening agent is a material with an ability to temporarily elastically bond the coils of the spring together. Thus, when retraction is initiated by pushing the actuator button, the bond between adjacent coils achieved by the dampening agent slows the initial opening of the coils from the tightly compressed state of the spring in the collapsed condition. As a result, the dampening agent preferably provides a slower initial acceleration and hence reduces splatter.

The apparatus may be used with a catheter telescoped over the piercing element and frictionally retained on the hub or barrel.

The retractable apparatus is packaged with the piercing element in a distal position relative to the barrel. The packaging preferably is configured to prevent inadvertent actuation. The apparatus then is removed from its packaging for use. Use commences by folding the wings into face-to-face engagement with one another and holding the folded wings between a thumb and forefinger. The health care worker then pulls the protector from the apparatus to expose the distal end of the piercing element. The distal end of the piercing element then is guided into a blood vessel or other targeted location. The fitting that may be provided at the proximal end of the tubing may be placed in communication with a source or reservoir for fluid in those instances where the apparatus is part of a fluid collection set or an infusion set. The ordering of these steps may vary depending on whether the set is used for fluid collection or for infusion. Upon completion of the medical procedure, the health care worker merely depresses the actuator button. The depression of the actuator button releases the actuator button from the actuating aperture and permits the spring to propel the piercing element proximally and into the shielded position within the barrel. The locking structure on the barrel prevents complete proximal separation of the piercing element from the barrel and prevents re-exposure of the used piercing element.

### Brief Description of the Drawings

FIG. 1 is an exploded perspective view of a fluid collection or infusion set in accordance with the invention.

FIG. 2 is a perspective view of the fluid collection set in its assembled condition.

FIG. 3 is an exploded perspective view of the needle assembly.

FIG. 4 is a perspective view of the needle assembly in its assembled condition.

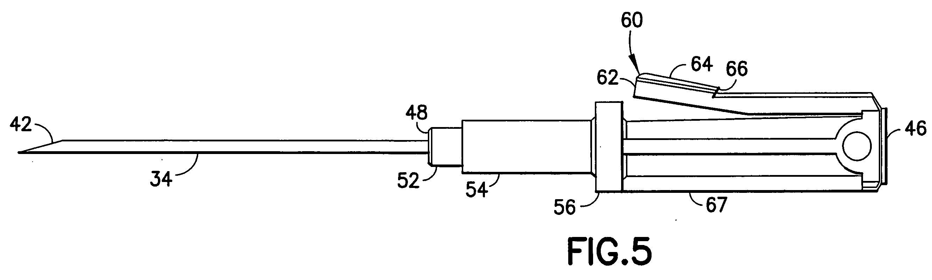

FIG. 5 is a side elevational view of the needle assembly.

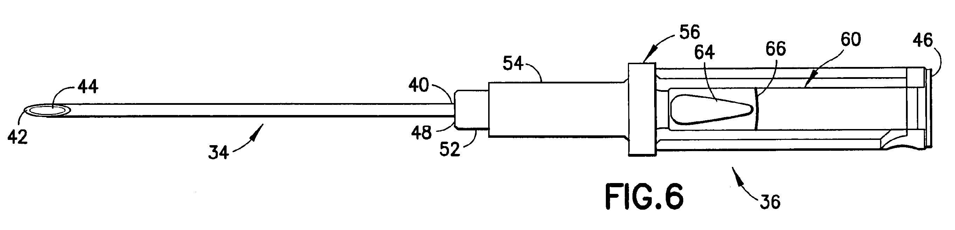

FIG. 6 is a top plan view of the needle assembly.

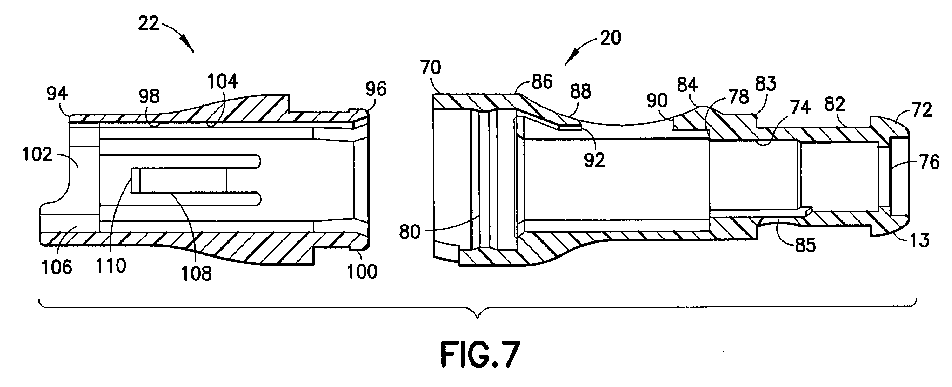

FIG. 7 is an exploded cross-sectional view of the barrel.

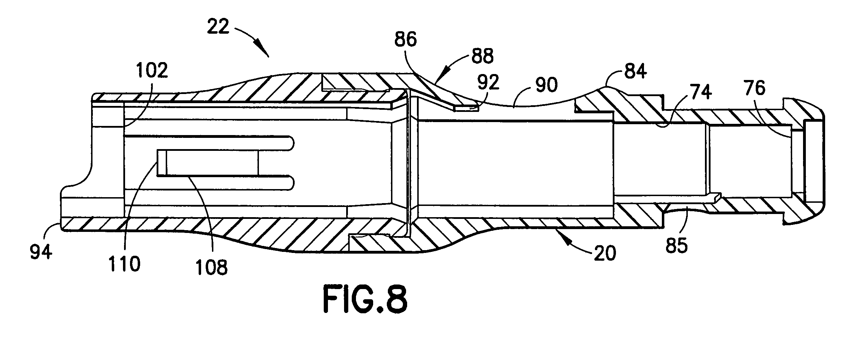

FIG. 8 is a longitudinal cross-sectional view of the barrel in its assembled condition.

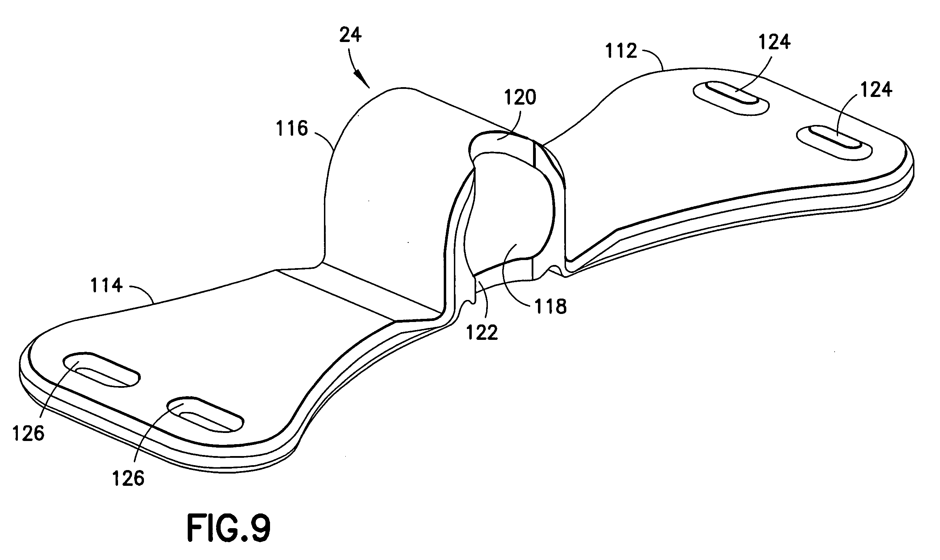

FIG. 9 is a perspective view of one embodiment of the wings.

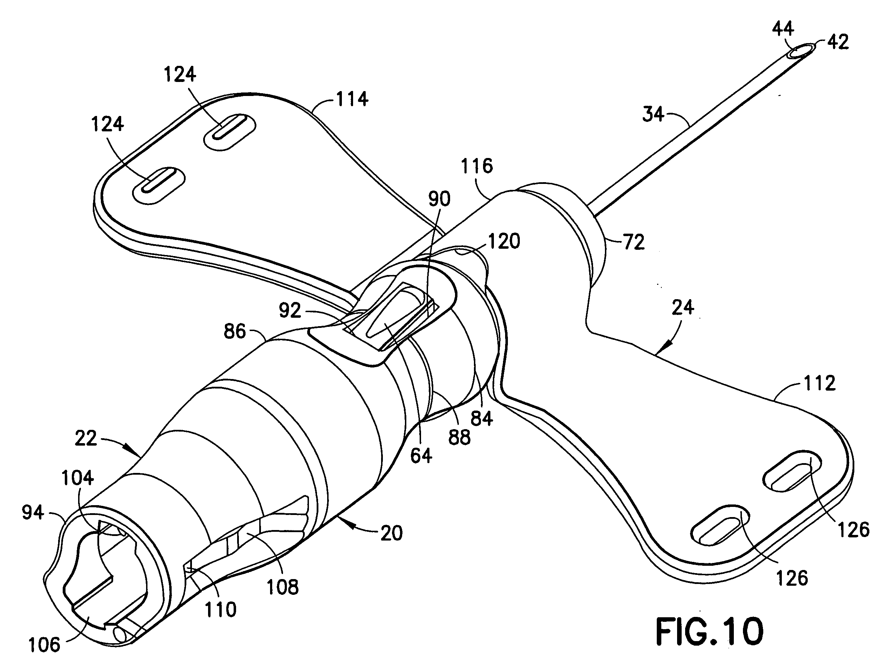

FIG. 10 is a perspective view of the retractable needle apparatus of the fluid collection set with the needle assembly in its distal position.

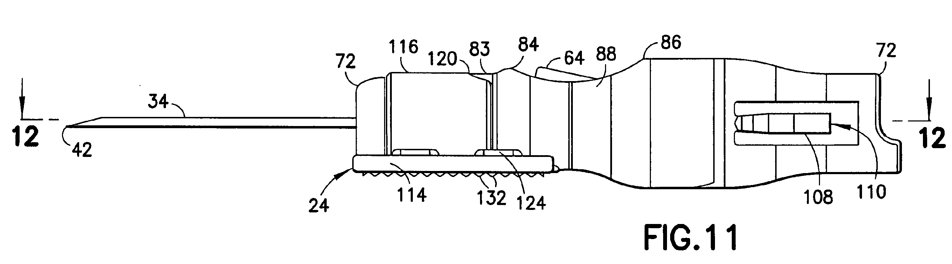

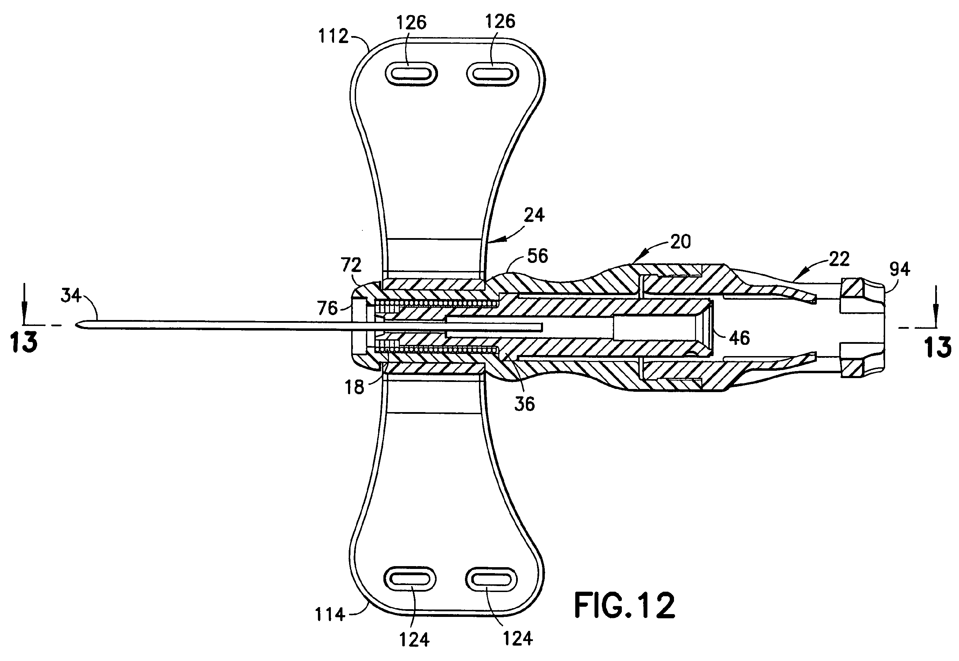

FIG. 11 is a side elevational view of the retractable needle apparatus shown in FIG. 12.

FIG. 12 is a cross-sectional view taken along line 12-12 in FIG. 11.

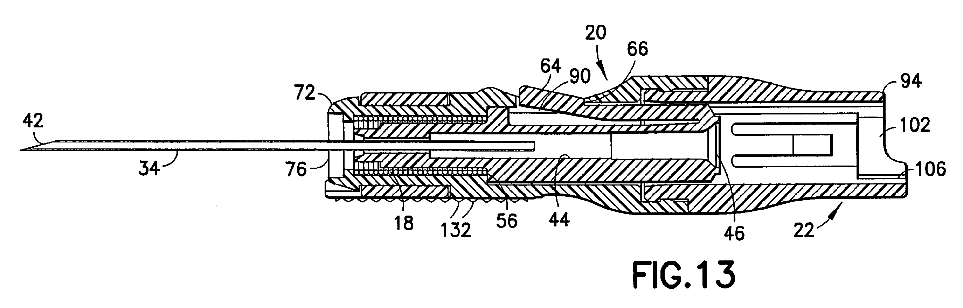

FIG. 13 is a cross-sectional view taken along line 13-13 in FIG. 12.

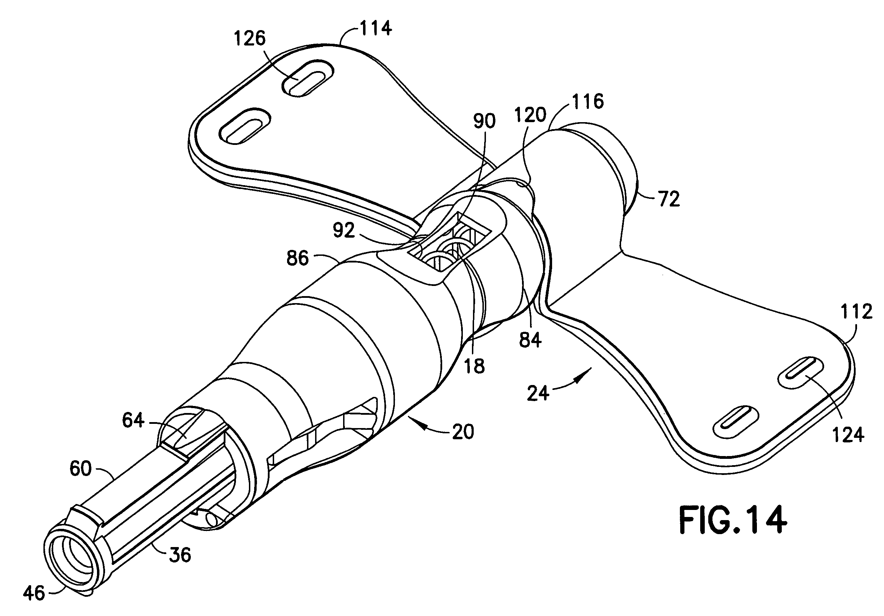

FIG. 14 is a perspective view similar to FIG. 10, but showing the needle assembly in the retracted position.

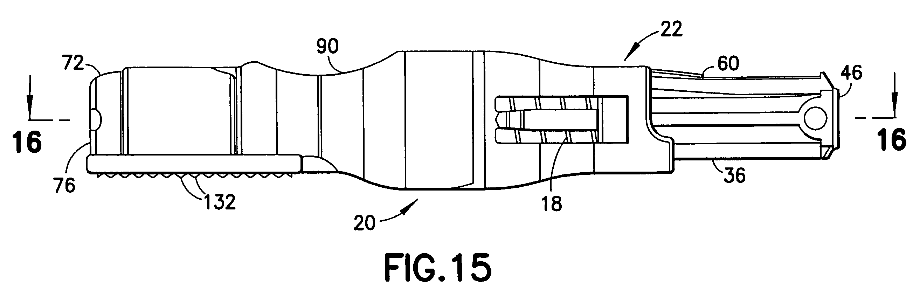

FIG. 15 is a side elevational view similar to FIG. 13 but showing the needle assembly in the retracted position.

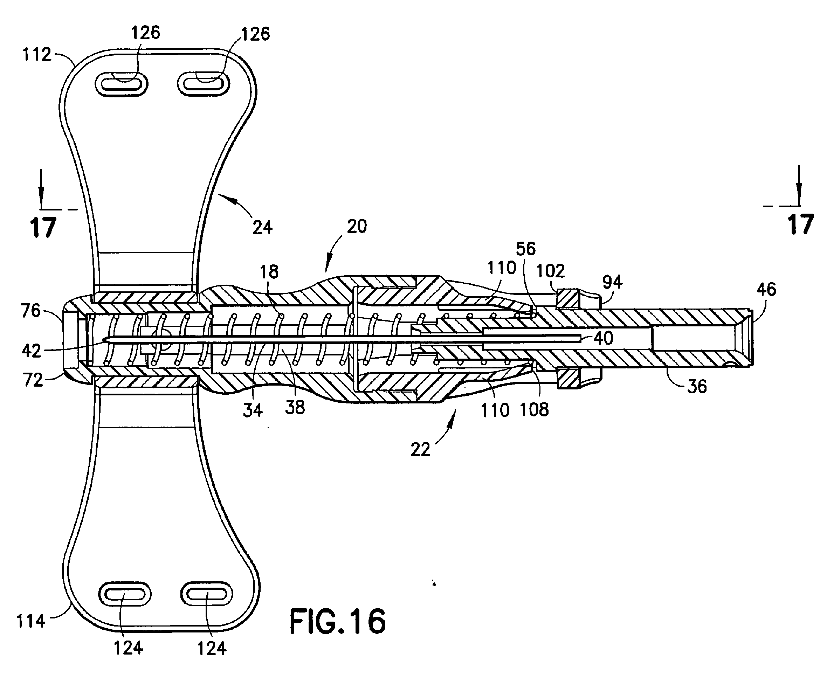

FIG. 16 is a cross-sectional view taken along line 16-16 in FIG. 15.

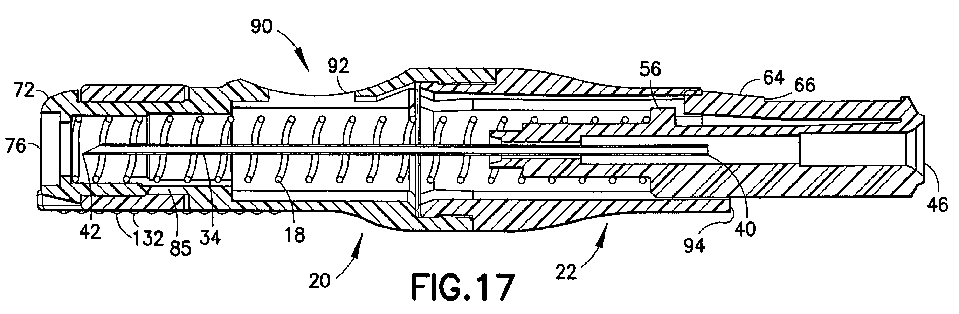

FIG. 17 is a cross-sectional view taken along line 17-17 in FIG. 16.

FIG. 18 is a perspective view similar to FIG. 10, but showing an additional embodiment of the wings.

FIG. 19 is a side elevational view of the embodiment in FIG. 18.

FIG. 20 is a cross-sectional view taken along line 20-20 in FIG. 18.

FIG. 21 is a side elevational view of the wings shown in FIGS. 18-20.

FIG. 22 is a perspective view of the apparatus used with a catheter.

FIG. 23 is a longitudinal cross-sectional view of the retractable apparatus of FIG. 22.

FIG. 24 is an exploded perspective view showing the piercing element prior to retraction, but after separation from the catheter.

### Detailed Description of the Invention

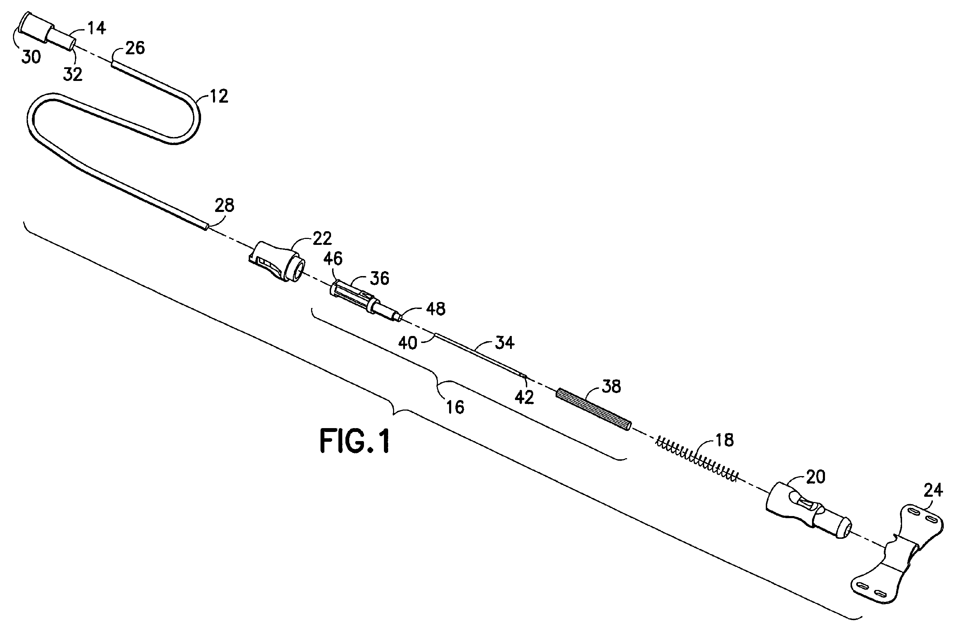

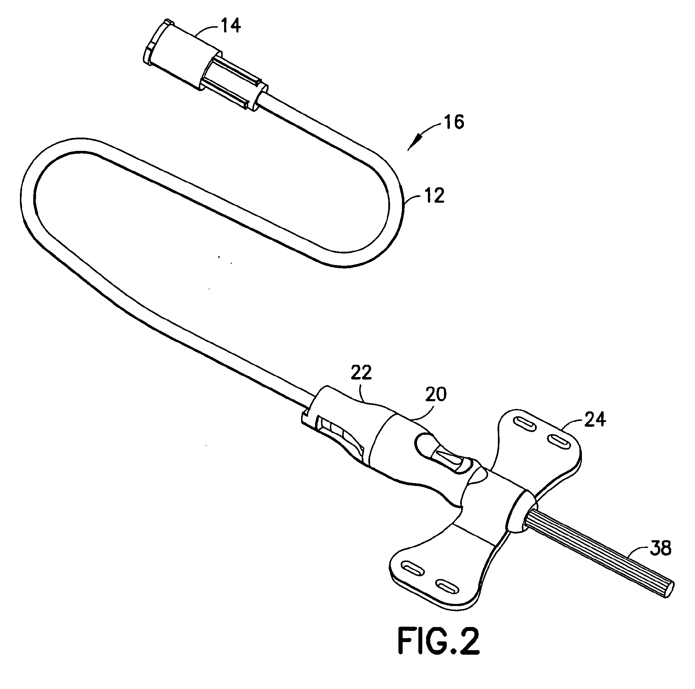

A fluid collection/infusion set in accordance with the subject invention is identified generally by the numeral **10** in FIGS. 1-3. Fluid collection/infusion set **10** includes a length of flexible plastic tubing **12,** a proximal fitting **14,** a needle assembly **16**, a spring **18** and a barrel assembly that comprises a front barrel **20**, a rear barrel **22** and a wing **24**.

Tubing **12** includes a proximal end **26**, a distal end **28** and a passage extending between the ends. Tubing **12** may be conventional intravenous tubing used in conventional blood collection sets or infusion sets.

Proximal fitting **14** is molded unitarily from a plastic material and includes a proximal end **30**, a distal end **32** and a passage extending between the ends. Portions of the passage adjacent distal end **32** are configured to telescope tightly over proximal end **26** of tubing **12** so that the passage through tubing **12** communicates with the passage through connector **14**. Proximal end **30** of fitting **14** defines a female luer connector that can be mated with an appropriate male luer connector to infuse a medication into a patient. The male luer connector may include a proximal needle cannula that can be placed in communication with an evacuated tube. In addition, the male luer connector may include an evacuated tube holder mounted to the male luer connector hub. Alternatively, a male luer connector at the distal end of a conventional prior art syringe can be connected directly to proximal fitting **14** for infusing a medication into the patient. In this instance, a separate male luer cap can be provided for closing proximal fitting **14**. Other fittings may be threadedly engaged with proximal fitting **14** in accordance with the specific intended use of collection/infusion set **10**. Additionally, proximal connectors of other configurations may be employed to achieve a particular objective. For example, fitting **114** in FIG. 1 is a non-patient needle assembly with a male luer hub **115**, a non-patient needle **116** and a non-patient sleeve **117** mounted over non-patient needle **116** and secured to male luer hub **115**. The non-patient sleeve **117** functions as a valve that permits multiple punctures of evacuated containers.

Needle assembly **16** includes a needle cannula **34**, a needle hub **36** and a needle protector **38**. Needle cannula **34** has a proximal end **40**, a distal end **42** and a lumen **44** extending between the ends. Distal end **42** of needle cannula **34** is beveled to a sharp tip.

Needle hub **36** is molded unitarily from a plastic material such as polycarbonate, polypropylene, polyethylene, acrylic, polystyrene and ABS. Preferably needle hub **36** is molded from a transparent or translucent material to enable observation of blood or other fluid flowing through needle hub **36**, such as by solvent bonding or welding. Needle hub **36** includes a proximal end **46**, a distal end **48** and a stepped passage **50** extending between the ends. Portions of passage **50** adjacent proximal end **46** are dimensioned to receive distal end **28** of tubing **12.** More particularly, distal end **28** of tubing **12** is telescoped into passage **50** of needle hub **36** and is bonded in position adjacent proximal end **46** of needle hub **36**. Portions of passage **50** adjacent distal end **48** of needle hub **36** are dimensioned for slidable receipt of proximal end **40** of needle cannula **34**.

External portions of needle hub **36** adjacent distal end **48** define a small diameter cylindrical tip **52**. An intermediate diameter cylindrical spring mounting section **54** extends proximally from small diameter cylindrical tip **52**, and a larger diameter cylindrical flange **56** extends outwardly at proximal end of spring mounting section **54.** Flange **56** defines a limit for proximal movement of spring **18** on needle hub **36** and a limit for distal movement of needle hub **36** relative to front barrel **20**.

An actuator arm **60** is cantilevered to extend outwardly and distally from proximal end **46** of needle hub **36**. The outward projection enables actuator arm **60** to function as a key that ensures and maintains a specific rotational orientation of needle hub **36** relative to front and rear barrels **20** and **22**. Additionally, actuator arm **60** and beveled tip **42** of needle cannula **34** are aligned symmetrically with one another. More particularly, a plane passing symmetrically through actuator arm **60** would also bisect the ellipse defined by beveled tip **42**. Alternatively, actuator arm **60** may be located on any side of needle hub **36**.

Actuator arm **60** includes a distal end **62** that is located proximally of flange **56.** Thus, flange **56** does not impede inward deflection of actuator arm **60.** Portions of actuator arm **60** proximally of distal end **62** define an actuator button **64** that projects radially outwardly on actuator arm **60**. The proximal end of actuator button **64** defines a locking edge **66** which is undercut relative to remaining portions of actuator arm **60** and oriented at an acute angle to the axis of needle hub **36**. A bottom stabilizing rib **67** extends axially along needle hub **36**. If needed, more than one stabilizing rib may be used.

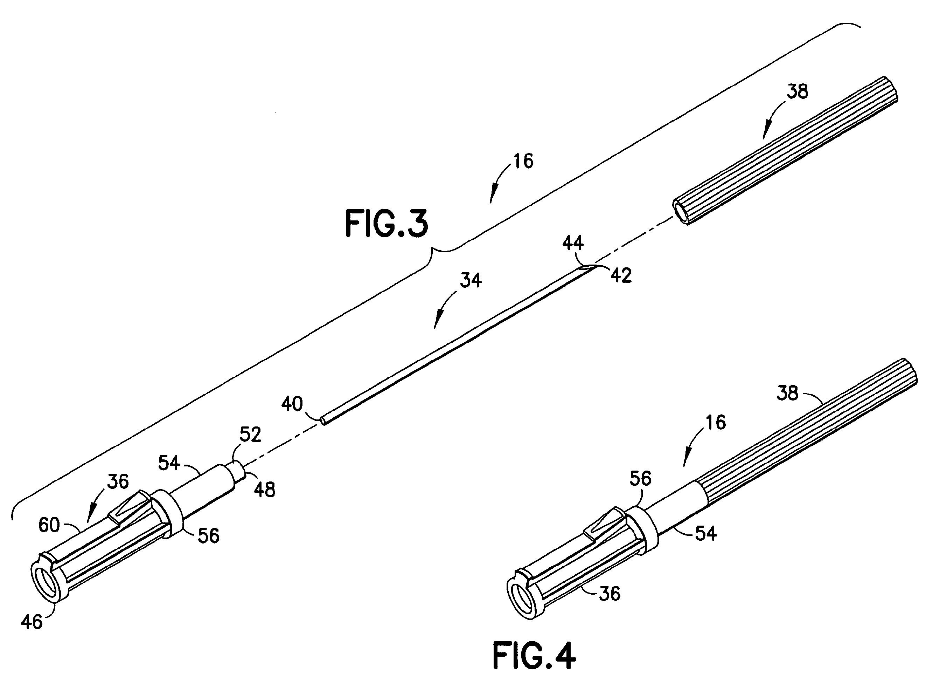

Needle protector **38** is a rigid cylindrical tube that provides the ability to extend past the projecting length of needle cannula **34** from distal end **72** of front barrel **20**. As shown in FIG. 3, needle protector **38** attaches to needle hub **36** and has a length that exceeds the projecting length of needle cannula **34** from needle hub **36**. Needle protector **38** defines an inside diameter approximately equal to the outside diameter of distal tip **52** of needle hub **36**. Additionally, needle protector **38** defines an outside diameter approximately equal to the outside diameter of spring mounting section **54** of needle hub **36**. Thus, as shown most clearly in FIG. 4, needle protector **38** can be telescoped over needle cannula **34** and frictionally retained on distal tip **52** of needle hub **36**. Additionally, in this mounted condition, spring mounting section **54** of needle hub **36** and needle protector **38** define a continuous and substantially uniform outside diameter. Alternatively, needle protector **38** may be retained frictionally on distal end **72** of front barrel **20** to further extend past the needle cannula **34**.

Spring **18** defines a helical coil with an inside diameter slightly greater than the outside diameter of needle protector **38** and spring mounting section **54** of needle hub **36**. Additionally, inside diameter of spring **18** is less than the outside diameter of flange **56** on needle hub **36**. Thus, flange **56** defines a limit to the range of telescoping movement of spring **18** over needle assembly **16**. The axial length of spring **18** is selected to conform with the desired range of movement of needle assembly **16** relative to front and rear barrels **20** and **22**. More particularly, the axial length of spring **18** in its expanded condition exceeds the distance between distal tip **42** of needle cannula **34** and flange **56** on needle hub **36**.

Front barrel **20** is a unitarily molded plastic structure with opposite proximal and distal ends **70** and **72**, and a passage **74** extending between the ends. Portions of passage **74** near distal end **72** define an inwardly extending annular distal flange **76** with an inside diameter less than the outside diameter of spring **18**. Thus, distal flange **76** defines a distal stop for spring **18** and enables spring **18** to be compressed within front barrel **20**. Passage **74** further has an annular step **78** proximally of distal flange **76.** Step **78** defines an inside diameter less than the outside diameter of flange **56** on needle hub **36**. Thus, step **78** defines a fixed limit for distal movement of needle hub **36** in front barrel **20**. Step **78** is spaced from distal flange **76** by a distance substantially equal to the compressed length of spring **18.** Thus, the section of passage **74** between distal flange **76** and step **78** effectively defines a spring housing. Passage **74** is defined further by an annular locking rib **80** near proximal end **70**. Locking rib **80** permits locked engagement of front and rear barrels **20** and **22** as explained herein.

The outer circumferential surface of front barrel **20** is defined by an annular wing-mounting undercut **82** near distal end **72**. Annular undercut **82** is provided with detents **83** for positioning wings **24** in a fixed rotational orientation on front barrel **20**. Undercut **82** of front barrel **20** may have a dampening agent injection port 85 for injecting a dampening agent into passage **74.** Port **85** then is covered by wings **24**.

Portions of the outer surface of front barrel **20** proximally of annular undercut **82** are flared outwardly to larger cross-sectional dimensions. However, the outer circumferential surface is necked down to define a reduced diameter portion that extends through at least approximately 270° around the circumference of front barrel **20.** Thus, front barrel **20** has a distal major diameter portion **84,** a proximal major diameter portion **86** and a minor diameter portion **88** therebetween. Minor diameter portion **88** of front barrel **20** includes an actuator aperture **90** extending therethrough and communicating with passage **74**. Actuating aperture **90** is dimensioned and configured to receive actuating button **64** and includes a locking edge **92** configured for engaging locking edge **66** of actuating button **64**. Actuating aperture **90** is positioned angularly at a central location on minor diameter portion **88**, and is aligned with projection **83** on undercut **82** to define a visually apparent top for an otherwise substantially symmetrical front barrel **20**. Step **78** is spaced from actuating aperture **90** by a distance equal to or slightly greater than the axial distance between distal end **62** of actuator arm **60** and the distal face of flange **56**. Thus, actuator button **64** is engaged in actuating aperture **90** when flange **56** of needle hub **36** substantially abuts step **78** of front barrel **20**. Additionally, the internal cross-sectional dimension of passage **78** adjacent to and proximal of locking edge **92** is substantially equal to or slightly less than the cross-sectional dimension of actuating arm **60** adjacent to and proximally of locking edge **66**. Hence, locked engagement is assured between locking edges **66** and **92** when needle hub **36** is moved distally in front barrel **20** a sufficient distance for flange **56** to substantially abut step **78**.

Rear barrel **22** also is a substantially tubular structure with a proximal end **94**, a distal end **96** and a passage **98** extending between the ends. Exterior portions of outer barrel **22** adjacent distal end **96** define an annular locking bead or ring **100**. Locking bead **100** is configured for snapped locked engagement with annular locking rib **80** in passage **74** of front barrel **20** to engage front and rear barrels **20** and **22** with one another. The engagement of front and rear barrels **20** and **22** can be made more permanent by adhesive bonding, welding, or by increasing the interference between annular locking rib **80** and locking bead **100**. Alternately, front barrel **20** and rear barrel **22** may be connected by threaded engagement where one of front or rear barrels **20** and **22** has external threads and the other of front and rear barrel **20** and **22** has internal threads. Thread pitch and location would be chosen to enable alignment of top and bottom axially extending channels **104** and **106**.

Proximal portions of passage **98** through rear barrel **22** are characterized by an inwardly extending proximal flange **102**. Proximal flange **102** has an inside diameter less than the outside diameter of flange **56** on needle hub **36**. Thus, proximal flange **102** limits proximal movement of needle hub **36** in rear barrel **22**.

Passage **98** of rear barrel **22** is characterized further by top and bottom axially extending channels **104** and **106** respectively. Top channel **104** is aligned with actuating aperture **90** and is dimensioned to slidably receive actuating arm **60** of needle hub **36**. Bottom channel **106** is dimensioned to slidably receive bottom stabilizing rib **67** of needle hub **36**. Portions of rear barrel **22** surrounding bottom channel **106** project proximally beyond top channel **104**. As a result, a greater axial length is provided for slidably receiving and supporting bottom stabilizing rib **67** of needle hub **36.** This additional support for bottom stabilizing rib **67** achieves a more desirable bearing ratio between the cross-sectional and axial dimensions for slidable engagement between needle hub **36** and barrels **20** and **22.** Accordingly, a more precise axial movement is achieved with less transverse shifting of needle hub **36**. The more precise axial movement enabled by the proximal extension surrounding bottom channel **106** substantially reduces splattering of residual fluid in needle cannula **34** during retraction.

Rear barrel **22** is characterized further by resiliently deflectable locking fingers **108** that are cantilevered proximally and inwardly from opposed locations on rear barrel **22** that are spaced from top and bottom channels **104** and **106** by approximately 90°. Each locking finger **108** includes a proximal end **110** that is spaced from proximal stop flange **102** by a distance equal to or slightly greater than the axial thickness of flange **56** on needle hub **36**. Hence, flange **56** can be trapped between the distal surface of stop flange **102** and locking figures **108** as explained below. Proximal ends **108** of locking figures **110** are spaced from one another by a distance less than the diameter of flange **56** on needle hub **36**.

Wings **24** are molded unitarily from an elastic material such as polyolefin, polyvinyl chloride or other such elastomeric polymers. Wings **24** include flexible side panels **112** and **114** and a tubular mount **116**. Tubular mount **116** includes an interior passage **118** that is dimensioned for snug engagement over under cut **82** on front barrel **20**. Additionally, mount **16** is formed with top and bottom notches **120** and **122** that are dimensioned to engage with detents **83** on front barrel **20** to ensure a preferred rotational orientation of wings **24**. Notches **120** and **122** are symmetrical about a plane that is perpendicular to panels **112** and **114**. Preferably, panels **112** and **114** are molded with a top surface that is relatively smooth. However, the top surface of panel **112** includes a pair of arcuate projections **124** at portions remote from tubular mount **116**. The top surface of panel **114** includes a pair of arcuate recesses **126** that are dimensioned, disposed and configured to receive projections **124** on panel **112** when panels **112** and **114** are folded so that the top surfaces thereof are in face-to-face engagement with one another. The interengagement of projections **124** with recesses **126** ensures that folded panels **112** and **114** function as a handle without slipping relative to one another. The bottom surfaces of panels **112** and **114** are provided with a plurality of tactile bumps **132**. Bumps **132** facilitate gripping of folded panels **112** and **114** between a thumb and forefinger of the user. The hinged movement of panels **112** and **114** about tubular mount **116** is facilitated by thinned regions at the connection of panels **112** and **114** with tubular mount **116**. The color of the wings **24** preferably designates the gauge of needle cannula **34.** Alternate embodiments where wings **24** have only one side panel **112** or **114** are contemplated to provide an alternate means to manipulate the needle assembly by the user.

Fluid collection set **10** is assembled by first mounting proximal end **40** of needle cannula **34** into passage **50** adjacent distal end **48** of needle hub **36.** Needle cannula **34** may be secured in this position by an adhesive, such as a heat curable or ultraviolet cured epoxy. As noted above, the orientation of the bevel that defines distal tip **42** of needle cannula **34** is important. Thus, needle cannula **34** is oriented such that the bevel at distal end **42** of needle cannula **34** and wings **126** arm **60** of needle hub **36** are symmetrical about a common plane. Orientation of wings **24** to distal end **42** of cannula **34** is guaranteed by relative orientation of actuator arm **60** and needle hub **36** with respect to front and rear barrels **20** and **22**. Needle assembly **16** is completed by telescoping protector **38** over needle cannula **34** sufficiently for frictional engagement on distal tip **52** of needle hub **36**. Alternately, protector **38** can be telescoped over needle cannula **34** by fictional engagement with front barrel **20.**

Distal end **28** of tubing **12** then is secured in proximal end **46** of needle hub **36**. Tubing **12** may be secured in this position by solvent bonding, adhesive bonding or welding.

Assembly continues by telescoping spring **18** over needle protector **38** and over spring mounting section **54** of needle hub **36.** Needle assembly **16** and spring **18** then are aligned and telescoped in a distal direction into front barrel **20**. This insertion requires actuator arm **60** and stabilizing rib **67** to align with channels **104** and **106**. Movement of needle hub **36** into front barrel **20** causes needle protector **38** to advance through and beyond distal end **72** of front barrel **20**. Additionally, actuator arm **60** is depressed sufficiently to clear portions of passage **74** immediately proximally of actuating aperture **90**. This distal movement causes spring **18** to collapse between distal flange **76** on front barrel **20** and flange **56** on needle hub **36**. As flange **56** of needle hub **36** approaches step **78** of front barrel **20**, actuator button **64** aligns with actuating aperture **90.** Thus, actuator arm **60** resiliently returns toward an undeflected condition and locking edge **66** of actuator button **64** engages locking edge **92** of actuating aperture **90.** As a result, needle assembly **16** is locked in its distal position in front barrel **20** with spring **18** secured in a compressed condition with significant stored energy. Wing **24** then is mounted over distal end **72** of front barrel **20.** Notches **120** and **122** of wing **24** are aligned with detents **83** on front barrel **20.** Thus, a snug fit of mount **116** of wing **24** is achieved with undercut **82** and detents **83** to hold wing **24** on front barrel **20** and to prevent rotation. In this mounted condition, panels **112** and **114** of wing **24** define a plane extending substantially normal to the plane of symmetry defined by the bevel at distal tip **42** of needle cannula **34** and actuator arm **60** of needle hub **36**. Assembly continues by threading proximal end **26** of tubing **12** through rear barrel **22**. Sufficient distal movement of rear barrel **22** along tubing **12** enables locked engagement of distal end **96** of rear barrel **22** within proximal end **70** of front barrel **20**. Fitting **14** then can be secured to proximal end **26** of tubing **12**.

When a viscous dampening agent is used, the passage **74** of front barrel **20**, the spring mounting section **54** of needle hub **36**, and the distal surface of the flange **56** on needle hub **36** define a chamber that constrains the preferred location of the dampening agent. An injection port **85** located within the sidewall of front barrel **20** is preferred for dispensing the viscous dampening agent into the chamber. Preferably, the dampening agent can be injected through a dispensing cannula that has a distal end shaped to fit within injection port **85.** It is also contemplated that the dampening agent can be applied to passage **74**, spring **18**, needle hub **36**, or any of the three components prior to assembly to produce an alteration to retraction speed or velocity.

The viscous dampening agent may be a silicone that functions to dampen the velocity of needle hub **36** relative to front barrel **20** and rear barrel **22**. The viscous dampening agent creates a resistance to slow the retraction of needle hub **36** and needle cannula **34**. A preferred dampening agent is a thixotropic gel, similar to the type of gel used as a separator gel in blood collection tubes. A thixotropic gel used as a dampening agent provides unique properties relative to spring **18**. In particular, the thixotropic gel exhibits the ability to temporarily and elastically bond adjacent coils of spring **18** together. Initiation of retraction releases the stored energy of spring **18,** and permits spring 18 to expand. The thixotropic gel creates resistance similar to silicone, and hence dampens the velocity of hub **36** and needle cannula **34.** However, unlike conventional silicone, the temporary bonding between adjacent coils achieved by the thixotropic gel provides a slower initial acceleration. The slower initial acceleration results in a significant reduction in splatter during retraction of needle cannula **34.**

Injection port **85** can be positioned on undercut **82** and can be sealed by placing wing **24** on and covering injection port **85**, thereby constraining the dampening agent to that portion of the spring **18** near the injection port **85**. Alternatively, it is understood that a dampening agent can be located at surfaces in slidable engagement between the needle hub **36** and front and rear barrels **20** and **22**. This would produce a viscous shearing boundary layer that also can alter the velocity and acceleration of needle hub **36** retraction.

Fluid collection or infusion set **10** is used by folding panels **112** and **114** of wing **24** toward one another and into face-to-face engagement so that projections **118** on upper surface of panel **112** are received in recesses **120** on the upper surface on panel **114** to prevent shifting of panels **112** and **114**. Tactile bumps **132** on the bottom surfaces of panels **112** and **114** then can be held securely in face-to-face engagement between a thumb and forefinger. Needle protector **38** then is separated from needle hub **36** to expose needle cannula **34**. In this condition, the plane defined by abutting surfaces of panels **12** and **14** of wing **24** will lie on the plane of symmetry of beveled distal tip **42** of needle cannula **34.** The health care worker then guides beveled distal tip **42** of needle cannula **34** into a targeted location on the patient and employs fitting **14** at proximal end **26** of tubing **12** for connection to an evacuated container or a source of fluid that will be infused into the patient. Upon completion of the medical procedure, the health care worker depresses actuator button **64** to withdraw needle cannula **34** proximally where front barrel **20** entirely encloses needle cannula **24.** In this regard, actuator button lies within the cross-sectionally reduced portion **88** of front barrel **20**, and hence is not susceptible to inadvertent actuation. However, the configuration of cross-sectionally reduced portion **88** is dimensioned to receive a tip of a forefinger that is intentionally directed toward actuator button **64**. Furthermore, the necked-down shape of front barrel **20** adjacent actuating aperture **90** provides a clear visual cue for the intended location of digital forces for depressing actuator button **64**.

Inwardly directed forces on actuator button **64** cause locking edge **66** of actuator button **64** to disengage from locking edge **92** of actuating aperture **90**. Hence, spring **18** is permitted to expand and propels needle assembly **16** proximally. Proximal movement of needle assembly **16** terminates when flange **56** abuts proximal stop flange **102** of rear barrel **22.** In this position, the entirety of needle cannula **38** is disposed safely within front and rear barrels **20** and **22**. The proximal movement of needle assembly **16** is guided axially by engagement of bottom stabilizing rib **67** in bottom channel **106**. Additionally, actuator button **64** travels in top channel **104** and biases needle assembly **16** toward bottom channel **106**, including portions of bottom channel **106** in proximal extension of rear barrel **22**. Hence, an effective bearing ratio is maintained to achieve merely axial movement, with a reduced probability of splatter as needle cannula **34** is accelerated proximally due to forces exerted by spring **38**.

As flange **56** of needle hub **36** approaches proximal stop **102**, flange **56** also will engage locking fingers **108**. Rearward movement of flange **56** causes an outward deflection of locking fingers **108.** However, when flange **56** abuts proximal stop **102,** locking fingers **108** resiliently return toward an undeflected condition and engage the distal face of flange **56**. Hence, a return movement of needle assembly **16** is prevented. Furthermore, the inwardly aligned orientation of locking fingers **108** substantially impedes any intentional outward deflection of locking fingers **108** that would permit a re-exposure of needle cannula 38. Hence, reuse of needle cannula **38** can be achieved only by a substantially complete destruction of the locking fingers in rear barrel **22**.

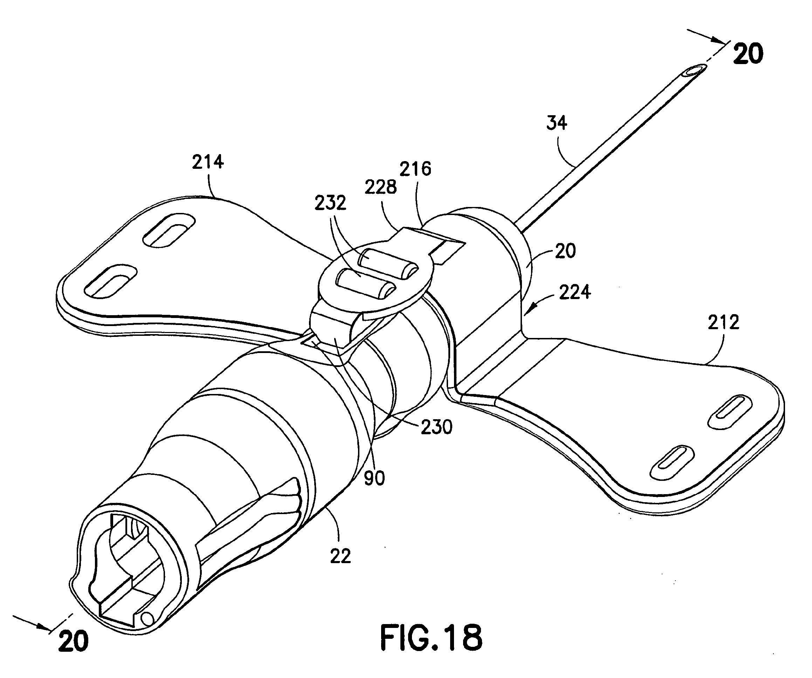

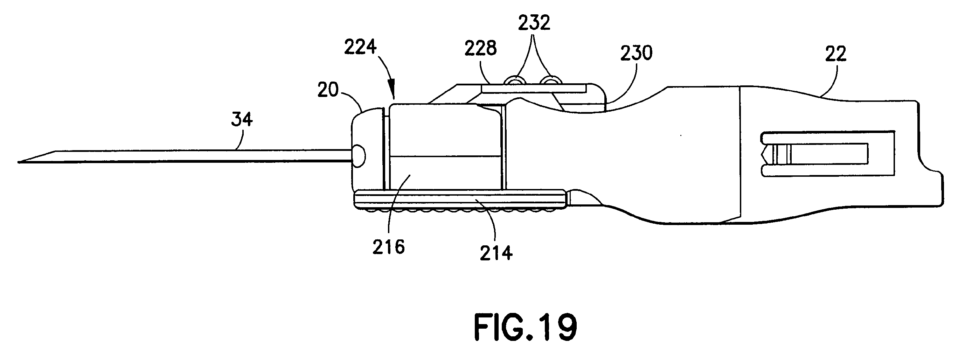

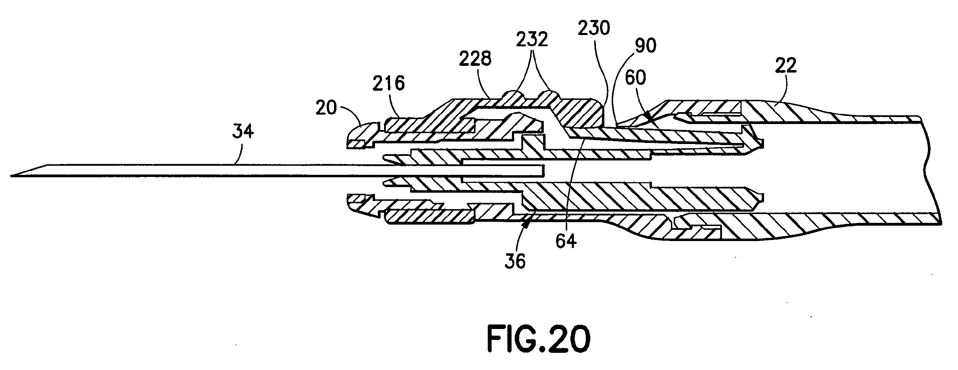

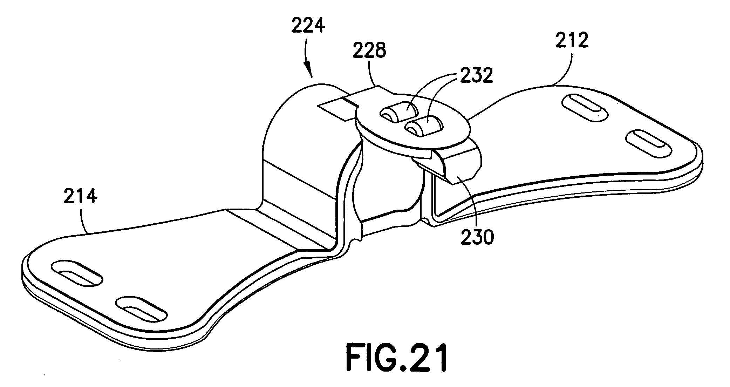

An alternate embodiment of the wings is identified by the numeral **224** in FIGS. 18-21. Wings **224** are similar to wings **24**, and include panels **212** and **214** that extend from a tubular mount **216.** However, wings **224** further have an actuating arm **228** extending proximally from tubular mount **216** and ending with a projection **230** that is disposed and dimensioned to register with actuating aperture **90** in front barrel **20** and with actuator button **64** of hub **36**. Upper surfaces of actuating arm **228** are wider than projection **230** and are provided with tactile bumps **232** projecting therefrom. Actuating arm **228** is flexible and provides the user an improved ability to indirectly depress actuator button **64** by depressing actuating arm **228** through aperture **90** and into actuating button **64.**

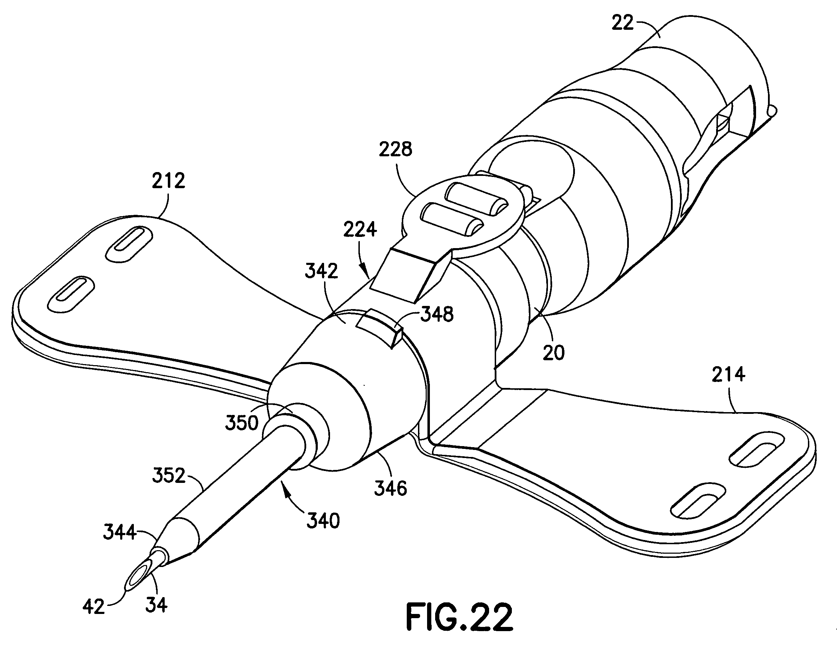

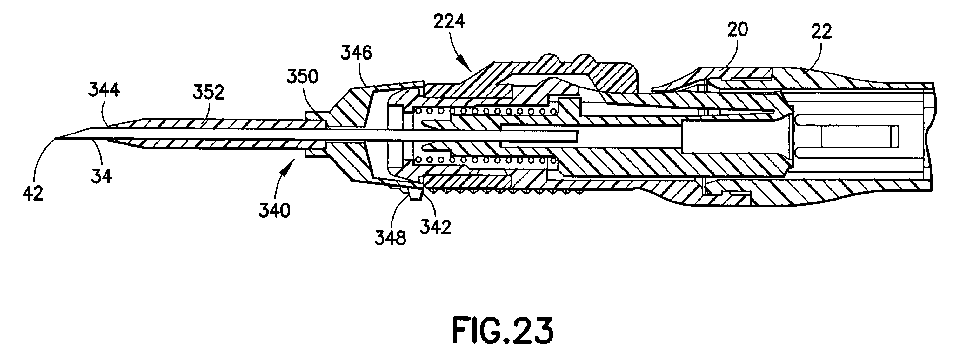

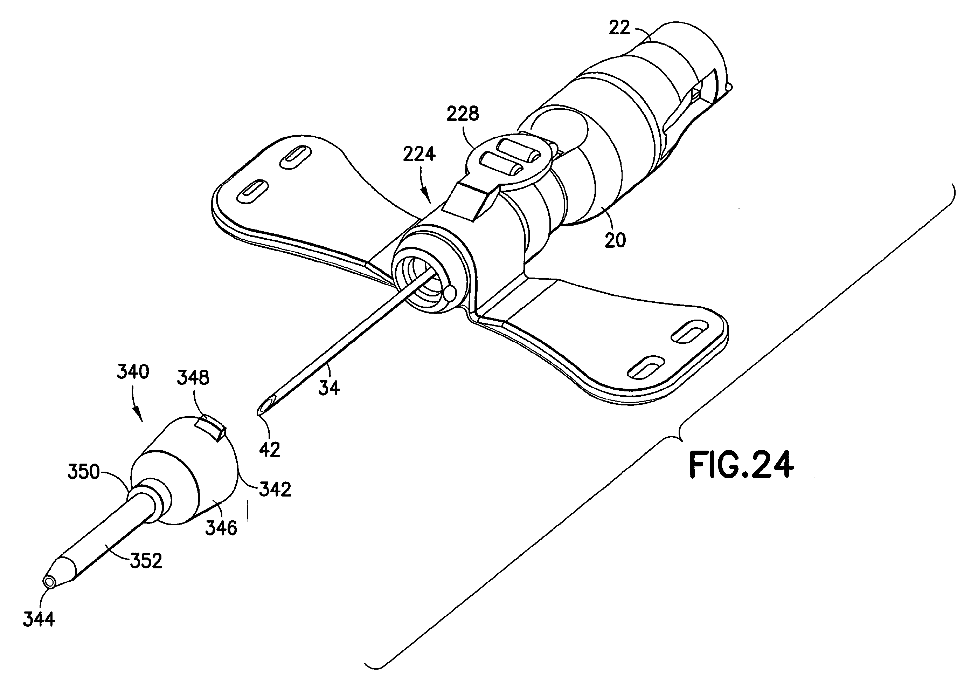

The preceding embodiments relate to fluid collection sets or fluid infusion sets. FIGS. 22-24 illustrate an embodiment of the invention that relates to a catheter. In particular, the embodiment of FIGS. 22-24 includes a needle assembly **16** that may be substantially identical to the needle assembly described and illustrated above. Needle assembly **16** is used with a coil spring **38** and is disposed for axial movement between front and rear barrels **20** and **22** as described above. In this embodiment, however, it is unnecessary to provide flexible plastic tubing or a proximal fitting. Rather, the embodiment of FIGS. 22-24 includes a catheter **340** with a proximal end **342** and a distal end **344**. Sections of catheter **340** adjacent proximal end **342** define a frustoconically generated mounting section **346** formed from a rigid plastic and dimensioned for frictional engagement over distal end **72** of front barrel **20**. Mounting section **346** is characterized by Luer lugs **348** extending outwardly thereon. Lugs **348** can be engaged threadedly with a Luer collar of a syringe or other medical device. Mounting section **346** includes a generally cylindrical distal end **350**. Catheter **340** further includes a generally tubular insertion section **352** inserted into cylindrical distal end **350** of mounting section **346** and secured therein by adhesive bonding, welding or the like. Insertion section **352** is formed from a material that is more pliable than mounting section **346**, such as polyurethane or silicone. Insertion section **352** has a cylindrical passage dimensioned for telescoping over needle cannula **34**. The outer surface of insertion section **352** is cylindrically generated. However, portions of insertion section **352** at distal end **344** of catheter **340** are conically tapered. Insertion section **352** defines a length selected so that distal end **344** of catheter **340** is disposed proximally of distal end **342** of needle cannula **34.** A needle protector (not shown) may be telescoped over both needle cannula **34** and insertion section **352** of catheter **340** and may be frictionally retained on cylindrical distal end **350** of mounting section **346.** The needle protector extends sufficiently to cover distal tip **42** of needle cannula **34**.

The embodiment of FIGS. 22-24 is used by first separating the protective cap to expose distal end **42** of needle cannula **34** and insertion section **352** of catheter **340.** Distal end **42** and needle cannula **34** then is guided into a targeted location on a patient and guides distal end **344** of catheter **340** into the patient. After the appropriate location has been accessed, the user exerts pressure on lugs **348** while pulling wing panels **112** and **114** proximally. Insertion section **352** of catheter **340** is squeezed closed as needle cannula **34** is withdrawn. This may be achieved with a hemostat or similar device. The Luer collar of a syringe then may be threadedly engaged with Luer lugs **348** on mounting section **346** of catheter **340** for delivering a liquid medication or other solution into a patient.

To summarize, the invention relates to a retractable safety needle comprising:
a needle hub having proximal and distal ends and a passage extending between said ends;
a needle cannula having a proximal end, a distal end and a lumen extending therebetween, said proximal end of said needle cannula being securely mounted in said passage at said distal end of said needle hub;
a resiliently deflectable actuator arm extending from said needle hub, an actuator button being formed on said actuator arm and projecting outwardly relative to said needle hub;
a barrel having a proximal end, a distal end and a passage extending between said ends, said needle hub being disposed in said passage of said barrel such that said needle hub, said needle cannula and said actuator arm are moveable relative to said barrel from a distal position where said needle cannula projects distally beyond said barrel and a proximal position where said needle cannula is disposed entirely in said barrel, said barrel having an actuating opening extending therethrough, said actuator button projecting into said actuating opening and engaging portions of said barrel adjacent said actuating opening when said needle hub is in said distal position, said actuator button being resiliently deflectable out of engagement with said actuating open; and
a spring disposed in said barrel and adjacent said needle hub for propelling said needle hub to said proximal position when said actuator button is deflected out of said actuating aperture,
wherein preferably said distal end of said needle cannula defines a substantially planar bevel, said bevel and said actuator arm being substantially symmetrical about a common plane,
wherein preferably the bevel and the actuator button face toward a common side of said retractable safety needle,
wherein preferably said actuator arm is cantilevered from said needle hub, said actuator arm having a base end unitary with said needle hub and a free end projected distally from said base end,
wherein preferably the actuator arm is a first actuator arm, the retractable safety needle further comprising a second actuator arm mounted to said barrel and projecting into a position for engaging and deflecting said first actuator arm,
further preferably comprising a pair of flexible wings mounted to said barrel and projecting transversely from said barrel, said wings defining a plane aligned substantially normal to a plane extending symmetrically through said first actuator arm, said second actuator arm being formed unitarily with said flexible wings,
further preferably comprising means for preventing re-exposure of said needle cannula after movement of said needle cannula to said proximal position relative to said barrel,
wherein preferably said means for preventing re-exposure comprises a plurality of resiliently deflectable locking fingers projecting proximally and inwardly from said barrel, said fingers being configured to lockingly engage a distal face of said needle hub when said needle hub reaches said proximal position for preventing re-exposure of said needle cannula,
wherein preferably the actuating window is disposed in a cross-sectionally reduced portion of said barrel such that portions of said barrel proximally and distally of said actuating window project radial distances greater than said actuator button for substantially preventing inadvertent actuation,
further preferably comprising a dampening material disposed in said barrel and surrounding at least portions of said spring and said needle hub for dampening movement of said barrel and said needle cannula during retraction, and thereby reducing splatter of material on said needle cannula,
wherein preferably said spring comprises a plurality of substantially adjacent coils, said dampening material being selected for exhibiting a temporary elastic bond between adjacent coils of said spring for providing a slow initial acceleration of said needle hub after deflecting said actuator button out of said actuating aperture, and
wherein preferably said dampening material is a thixotropic gel.

The invention also relates to the use of a thixotropic gel as a dampening agent for dampening a spring driven shielding of a needle cannula.

## Claims

1. A retractable safety needle comprising:
a needle hub (36) having proximal and distal ends and a passage extending between said ends;
a needle cannula (34) having a proximal end; a distal end and a lumen extending therebetween, said proximal end of said needle cannula (34) being securely mounted in said passage at said distal end of said needle hub (36);
a resiliently deflectable actuator arm (60) extending from said needle hub (36), an actuator button being formed on said actuator arm (60) and projecting outwardly relative to said needle hub (36);
a barrel (20, 22) having a proximal end, a distal end and a passage extending between said ends, said needle hub (36) being disposed in said passage of said barrel (20, 22) such that said needle hub (36), said needle cannula (34) and said actuator arm (60) are moveable relative to said barrel (20, 22) from a distal position where said needle cannula (34) projects distally beyond said barrel (20, 22) and a proximal position where said needle cannula (34) is disposed entirely in said barrel (20, 22), said barrel (20, 22) having an actuating opening extending therethrough, said actuator button projecting into said actuating opening and engaging portions of said barrel (20, 22) adjacent said actuating opening when said needle hub (36) is in said distal position, said actuator button being resiliently deflectable out of engagement with said actuating open;
a spring (18) disposed in said barrel (20, 22) and adjacent said needle hub (36) for propelling said needle hub (36) to said proximal position when said actuator button is deflected out of said actuating aperture; and
wherein said barrel (20, 22) includes an inwardly extending proximal flange (102) extending into said passage (98) at said proximal end of said barrel (20, 22) for engaging the flange (56) of the needle hub (36) and limiting proximal movement of said needle hub (36).

2. The retractable safety needle of Claim 1, wherein said distal end of said needle cannula (34) defines a substantially planar bevel, said bevel and said actuator arm (60) being substantially symmetrical about a common plane.

3. The retractable safety needle of Claim 2, wherein the bevel and the actuator button face toward a common side of said retractable safety needle.

4. The retractable safety needle of any of Claims 1-3, wherein said actuator arm (60) is cantilevered from said needle hub (36), said actuator arm (60) having a base end unitary with said needle hub (36) and a free end projected distally from said base end.

5. The retractable safety needle of any of Claims 1-4, wherein the actuator arm (60) is a first actuator arm (60), the retractable safety needle further comprising a second actuator arm (228) mounted to said barrel (20) and projecting into a position for engaging and deflecting said first actuator arm (60).

6. The retractable safety needle of Claim 5, further comprising a pair of flexible wings mounted to said barrel and projecting transversely from said barrel (20), said wings defining a plane aligned substantially normal to a plane extending symmetrically through said first actuator arm (60), said second actuator arm (228) being formed unitarily with said flexible wings.

7. The retractable safety needle of any of Claims 1-6, further comprising means for preventing re-exposure of said needle cannula (34) after movement of said needle cannula (34) to said proximal position relative to said barrel (20).

8. The retractable needle assembly of Claim 7, wherein said means for preventing re-exposure comprises a plurality of resiliently deflectable locking fingers projecting proximally and inwardly from said barrel (20), said fingers being configured to lockingly engage a distal face of said needle hub (36) when said needle hub (36) reaches said proximal position for preventing re-exposure of said needle cannula (34).

9. The retractable safety needle of any of Claims 1-8, wherein the actuating window is disposed in a cross-sectionally reduced portion of said barrel (20, 22) such that portions of said barrel (20, 22) proximally and distally of said actuating window project radial distances greater than said actuator button for substantially preventing inadvertent actuation.

10. The retractable safety needle of any of Claims 1-9, further comprising a dampening material disposed in said barrel (20, 22) and surrounding at least portions of said spring (18) and said needle hub (36) for dampening movement of said barrel (20, 22) and said needle cannula (34) during retraction, and thereby reducing splatter of material on said needle cannula (34).

11. The retractable safety needle of Claim 10, wherein said spring (18) comprises a plurality of substantially adjacent coils, said dampening material being selected for exhibiting a temporary elastic bond between adjacent coils of said spring (18) for providing a slow initial acceleration of said needle hub (36) after deflecting said actuator button out of said actuating aperture.

12. The retractable safety needle of Claim 10 or 11, wherein said dampening material is a thixotropic gel.

13. The use of a thixotropic gel as a dampening agent for dampening a spring driven shielding of a needle cannula (34).
